(19) Europäisches Patentamt
European Patent Office
Office européen des brevets

(11) EP 2 284 275 A1

# (12) EUROPEAN PATENT APPLICATION

(43) Date of publication:
**16.02.2011 Bulletin 2011/07**

(51) Int Cl.:
***C12Q 1/00*** *(2006.01)*

(21) Application number: **09167572.8**

(22) Date of filing: **10.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Sensile Pat AG**
**4614 Haegendorf (CH)**

(72) Inventors:
- **Lavanant, Laurent**
  **74500 Evian les Bains (FR)**
- **Klok, Harm-Anton**
  **1025 St-Sulpice (CH)**

(74) Representative: **reuteler & cie SA**
**Chemin de la Vuarpillière 29**
**1260 Nyon (CH)**

## (54) Stimuli Responsive Membrane

(57) There is provided a glucose responsive membrane comprising a nanoporous support substrate and a layer of a glucose responsive hydrogel attached to a surface of the nanoporous substrate. There are also provided methods for the preparation of the glucose responsive membrane and a medical device for the monitoring or regulation of glucose levels in a patient comprising the membrane.

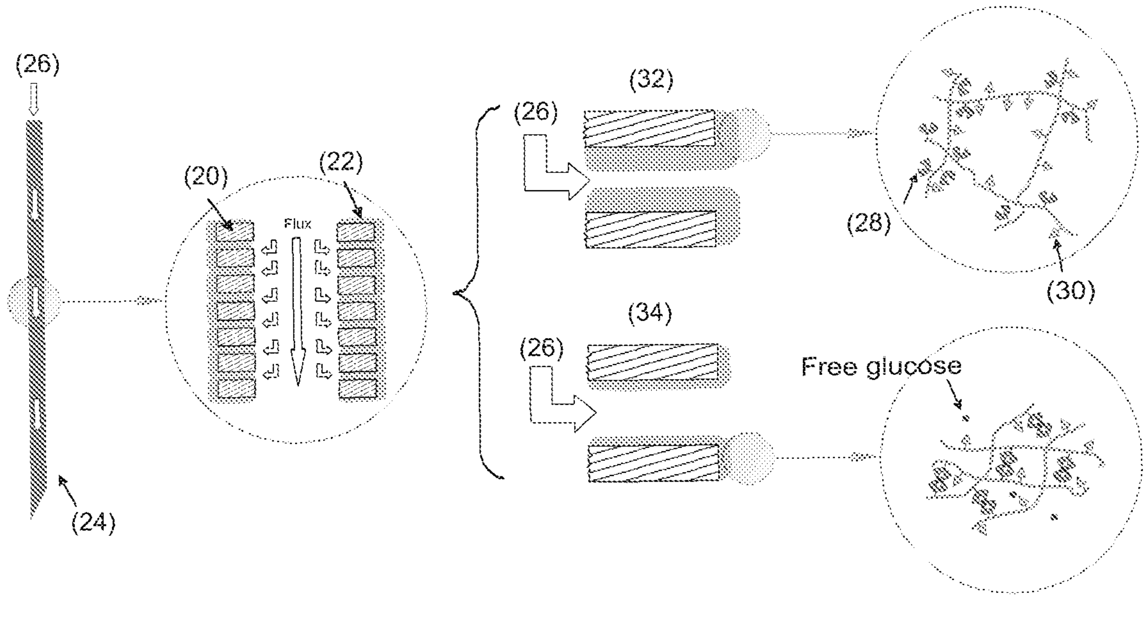


**Figure 2**

EP 2 284 275 A1

## Description

The present invention relates to the field of porous membranes, more particularly to the design and manufacture of porous membranes which are responsive to external stimuli, and to blood analyte monitoring and drug delivery devices comprising the membranes, in particular for the monitoring of glucose and for the treatment of patients with diabetes.

The regular or continuous measurement of an analyte concentration is necessary in the control or therapy of many conditions, such as diabetes. For instance, diabetic patients may require measurement of their blood glucose level several times a day, in order to appropriately adapt the administration of insulin. More measurements of the blood glucose level allow for drug administration regimes which regulate the blood glucose level of the diabetic patient more precisely, i.e. the fluctuations of the blood glucose level may be kept within a physiological range. Hence, it is crucial for a successful treatment of diabetic patients to obtain accurate, undelayed, and continuous information about the blood glucose level.

Various different medical devices have been proposed for the monitoring of blood glucose levels. Most conventionally used blood glucose monitors make use of chemical test strips which work on electro-chemical principles, whereby the patient withdraws a drop of blood for each measurement, generally requiring uncomfortable finger pricking methods. In order to avoid the pain caused by finger pricking and to allow more frequent, or continuous, control of glycaemia a variety of implantable sensors, including transdermal or subcutaneous sensors, are being developed for continuously detecting and/or quantifying blood glucose values. Glucose sensors for frequent or continuous glucose monitoring based on electrochemical, viscosimetric, or optical sensors have been widely investigated.

Different medical devices intended for the treatment of patients with diabetes have previously been described: Separate glucose sensors (e.g. electrochemical, viscosimetric, or optical sensors); separate medication delivery devices (e.g. insulin pumps and insulin pens); as well as so-called closed loop systems integrating glucose sensor and medication delivery, which ideally mimic the function of the pancreas, i.e. medication capable of controlling blood glucose level is released subject to blood glucose concentration.

Although a number of closed loop systems have been investigated, as yet none has been successfully developed for practical usage in patients.

To date most development in closed loop systems has related to so-called two port systems which consist of at least two separated units, connected through an electronic interface. For instance in patent application US 2006/0224141 there is described a system, in which the analyte monitoring unit is separated from the medication infusion unit. The analyte sensor is based on using electrodes in order to determine a change in electric resistance subject to a change of the analyte concentration. A semi-closed loop system is described in patent application WO 03047426A1, where an at least partially implanted glucose sensor is in communication with an injection pen, whereas the user can adjust the dose to be injected based on the glucose concentration measured by the glucose sensor. Such two port systems require the insertion of catheter or needle for insulin delivery at a different skin site to that of the glucose sensor. This spatial separation results in patient discomfort and reduces patient acceptability of the closed loop system.

WO 89/01794 discloses an implantable glucose sensor for a one port integrated drug delivery system. The sensor includes a liquid infusate, which is put under pressure and flows through a catheter. One section of the catheter contains a microporous membrane, where the concentration of the glucose present in the infusate is equilibrated with a response time between several minutes up to one hour. The equilibrated infusate then flows through a chemical valve which consists of a hydrogel matrix containing concanavalin A, and dextran molecules. The matrix in the chemical valve changes its solute permeability subject to the glucose concentration present in the infusate, thus regulating the amount of infusate flowing into the body of a patient.

When the system, as disclosed in WO 89/01794, is employed to solely monitor the concentration of glucose in the surrounding medium, the catheter contains an additional glucose sensor, such as an enzyme electrode, a fuel cell, or an affinity sensor, whereas the chemical valve is not present. Further proposed is a stand-alone sensor, in which the pressure in the infusate is determined before and after the infusate has passed the chemical valve, whereas the pressure-drop across the chemical valve is inversely proportional to the glucose concentration in the equilibrated infusate.

In order to control the blood glucose level in a patient with diabetes, it is necessary to obtain results quickly in order to adjust the delivery of drugs. That is why response times of components within the glucose sensor are a crucial factor for a successful drug delivery program. If, as described in WO 89/01794, an equilibration region has a response time of up to one hour, and a hydrogel matrix contained in a chemical valve has an additional response time, the drug administration is adjusted to a blood glucose value that is no longer present in the patient, and thus the regulation of the patient’s blood glucose level will not be optimal.

Further where, as in W089/0174, the hydrogel matrix is in a fluent state, i.e. new components (such as dextran molecules) that arrive with fresh infusate replace components that are washed away with the infusate into the patient’s body, then components that do not contribute to the treatment, or even are toxic such as is the case for concanavalin A, may enter the patient’s body. Moreover, the matrix is likely to change characteristics over time, as the replacement of new components may not take place in an evenly distributed manner. For instance, clusters are likely to occur at the infusate entry site(s) of the matrix where the infusate with new components arrives at first.

A number of hydrogels have been investigated for potential application in glucose concentration measurement (see for instance: T. Miyata, T. Uragami, K. Nakamae Adv. Drug Deliver. Rev. 2002, 54, 79. ; Y. Qiu, K. Park Adv. Drug. Deliver. Rev. 2001, 53, 321. ; S. Chaterji, I. K. Kwon, K. park Prog. Polym. Sci. 2007, 32, 1083. ; N. A. Peppas J. Drug Del. Sci. Tech. 2004, 14, 247-256). Hydrogels are cross-linked polymeric matrices that absorb large amounts of water and swell. These materials may be physically and chemically cross-linked to maintain their structural integrity. Hydrogels can be sensitive to the conditions of the external environment if they contain active functional groups. The swelling behavior of these gels may be dependent on for instance pH, temperature, ionic strength, solvent composition, or other environmental parameters. These properties have been used to design stimuli responsive or "intelligent" hydrogels such as glucose-sensitive polymeric systems. (see for instance: G. Albin, T.A. Horbett, B.D. Ratner, J. Controlled Release, 1985, 2, 153. ; K. Ishihara, M. Kobayashi, I. Shinohara Polymer J. 1984, 16, 625)

Among the different types of hydrogel which have been proposed, three main types of hydrogel have been investigated:

Glucose oxidase-loaded hydrogels:

The combination of a pH sensitive hydrogel with the enzyme glucose oxidase (GOD) has been investigated for the design of glucose responsive hydrogels. Glucose is enzymatically converted by GOD to gluconic acid which lowers the pH of the environment. The enzyme GOD has been combined to different types of pH sensitive hydrogels. In general for hydrogels that contain polycations, such as poly(N,N'-diethylaminoethyl methacrylate), the lowering of pH leads to hydrogel swelling due to the protonation of the N,N'-diethylaminoethyl side chain. When a hydrogel swells, molecules diffuse more easily through the hydrogel when compared to the collapsed state. Whereas, if the hydrogel contains polyanions, such as poly(methacrylic acid), the hydrogel swells at high pH value due to electrostatic repulsion among the charges on the polymer chains. After lowering of the pH, the polymer chains of the hydrogel collapse due to the protonation of the methacrylic acid side chains which reduces the electrostatic repulsion between the polymer chains. (Y. Ito, M. Casolaro, K. Kono, I. Yukio J. Controlled Release 1989, 10, 195)

Lectin-loaded hydrogels:

Another approach to design glucose responsive hydrogels consists in combining glucose containing polymers with carbohydrate-binding proteins, lectins, such as Concanavalin A (Con A). The biospecific affinity binding between glucose receptors of Con A and glucose containing polymers leads to the formation of a gel capable of reversible sol-gel transition in response to free glucose concentration. A variety of natural glucose containing polymers have been investigated such as polysucrose, dextran, and glycogen (see for instance : M. J. Taylor, S. Tanna, J. Pharm. Pharmacol. 1994, 46, 1051 ; M. J. Taylor, S. Tanna, P. M. Taylor, G. Adams, J. Drug Target. 1995, 3, 209 ; S. Tanna, M. J. Taylor, J. Pharm. Pharmacol. 1997, 49, 76 ; S. Tanna, M. J. Taylor, Pharm Pharmacol. Commun. 1998, 4, 117 ; S. Tanna, M. J. Taylor, Proc. Int. Symp. Contr. Rel. Bioact. Mater. 1998, 25, 737B. ; S. Tanna, M. J. Taylor, G. Adams, J. Pharm. Pharmacol. 1999, 51, 1093). Additionally, some synthetic polymers with well defined saccharide residues such as poly (2-glucosyloethyl methacrylate) (PGEMA) have been investigated. (K. Nakamae, T. Miyata, A. Jikihara, A. S. Hoffman J. Biomater. Sci. polym. Ed. 1994, 6, 79.)

Phenylboronic acid based hydrogels:

The fabrication and handling of glucose responsive hydrogels that incorporate proteins is difficult due to the instability of biological components. As an alternative approach, investigation has been made on synthetic hydrogels that contain phenylboronic acid (PBA) moieties.

Phenylboronic acid and its derivatives form complexes with polyol compounds, such as glucose, in aqueous solution. It has been shown that these Lewis acids can reversibly bind the cis-1,2- or -1,3-diols of saccharides covalently to form five- or six- membered rings ( C. J. Ward, P. Patel, T. D. James, Org. Lett. 2002, 4, 477.) The complex formed between phenylboronic acid and a polyol compound can be dissociated in the presence of a competing polyol compound which is able to form a stronger complex with the phenylboronic acid. Following this idea, the competitive binding of phenylboronic acid with glucose and poly(vinyl alcohol) has been investigated for the construction of a glucose-sensitive material. For example, the competitive binding of the PBA moieties of poly(N-vinyl-2-pyrrolidone)-co-poly(3-(acrylamido) phenylboronicacid) copolymer with glucose and poly(vinyl alcohol) (S. Kitano, Y. Koyama, K. Kataoka, T. Okano, Y. Sakurai, J. Controlled Release 1992, 19, 161).

In aqueous medium Phenylboronic acid compounds exist in equilibrium between an uncharged and a charged form. Only the charged phenylborates form a stable complex with glucose, whereas unstable complex are obtained between glucose and the uncharged form. When the concentration of glucose increases, the total amount of charged PBA moieties increases and the number of uncharged groups decreases which has a dramatic effect on the solubility

of the polymer in water (K. Kataoka, H. Miyazaki, T. Okano, Y. Sakurai, Macromolecules 1994, 27, 1061). This change in solubility has been investigated for the development of a glucose sensitive system. For example, PBA has been copolymerized with temperature sensitive polymer such as N-isopropylacrylamide (NIPAM) in order to obtain a polymer with a glucose sensitive low critical solution temperature (LCST) (T. Aoki, Y. Nagao, K. Sanui, N. Ogata, A. Kikuchi, Y. Sakurai, K. Kataoka, T. Okano, Polym. J. 1996, 28, 371). In this system a change in glucose concentration induces a change in the LCST of the hydrogel which, at a fixed temperature, will induce swelling/collapse of the hydrogel matrix.

Despite promising results, the systems described above cannot be used for application in in-vivo monitoring of glucose concentration for two important reasons:

1) Physiological condition: Reversible binding of phenylboronic acid with polyol was not achieved at physiological conditions (temperature, ionic strength and pH values).

2) Selectivity and interfering molecules: The binding of phenylboronic acid is not selective. Indeed, phenylboronic acids can form complexes with any saccharides possessing cis-1,2- or -1,3-diols (such as glucose, fructose and galactose). In healthy individuals glucose is normally present in the range 4-8 mM while fructose and galactose, the most abundant sugars after glucose, are usually present in physiological fluids at sub-mmol levels (R. Badugu, J. R. Lakowicz, C. D. Geddes, Analyst 2004, 129, 516). However, phenylboronic acids have a much greater affinity for fructose than glucose, (J. P. Lorand, J. O. Edwards, J. Am. Chem. Soc. 1959, 24, 769) a feature that may affect the accuracy of glucose measurement. Additionally, the presence of lactate is known to interfere with phenylboronic acid based hydrogels.

Some formulations of hydrogels with phenylboronic acid moieties have been recently investigated with the aim to improve the selectivity of the hydrogel and/or the better reversibility at physiological conditions. It has been found that the presence of basic groups, such as amines, in the neighbourhood of the PBA moieties allows the formation of stable complexes with glucose at physiological pH. This is due to basic amino groups which might coordinate to boron atoms to stabilize the tetrahedral form of the boronic acid moiety (see for instance: Kikuchi A, Suzuki K, Okabayashi O, Hoshino H, Kataoka K, Sakurai Y, Okano T: Glucose-sensing electrode coated with polymer complex gel containing phenylboronic acid. Analytical Chemistry 1996, 68, 823-828).

In another formulation, presented by Pritchard in 2006 (G. J. Worsley, G. A. Tourniaire, K. E. S. Medlock, F. K. Sartain, H. E. Harmer, M. Thatcher, A. M. Horgan, J. Pritchard Clinical Chem. 2007, 53, 1820-1826, a tertiary amine monomer (N-[3-(dimethylamino)propyl]-acrylamide) was copolymerized with 3-acrylamidophenylboronic acid to give a glucose responsive hydrogel with a specific affinity for glucose. In this case, an increase of the glucose concentration induces a contraction of the gel. The most probable explanation for the observed contraction is cross-linking of two neighboring boronic acid receptors with favorable stereochemistry by glucose to give a bis-boronateglucose complex. A film of this glucose responsive hydrogel has been loaded with light sensitive crystals of AgBr to design a holographic glucose sensor shown to have ability to detect glucose in human plasma conditions. (See for instance : S. Tanna, T. S. Sahota, J. Clark, M. J. Taylor J. Drug Target. 2002, 10 411; S. Tanna, T. Sahota, J. Clark, M. J. Taylor, J. Pharm. Pharmacol. 2002, 54, 1461).

One solution that has been proposed to provide selectivity to glucose at physiological pH and obviate lactate interference consists of designing PBA derivatives with lower pKa. Following this idea, 2-acrylamidophenylborate (2APB) has been designed and used to fabricate a holographic sensor, which displayed significant response to glucose with little interference from lactate, and with no dependence on pH in the physiological range (see for instance: Yang XP, Lee MC, Sartain F, Pan XH, Lowe CR: Designed boronate ligands for glucose-selective holographic sensors. Chemistry-a European Journal 2006, 12, 8491-8497). This could be explained by the formation of weak B-O intermolecular interactions, conferring a tetrahedral conformation at the boron centre through the neighbouring effect of the ortho group.

WO 2004/081624 describes a class of phenylboronic acid derivatives wherein the phenyl group comprises one or more substituent which via an electronic effect promotes formation of the more reactive tetrahedral geometry about the boron atom. According to WO 2004/081624 the substituents(s) may be electron withdrawing groups which, by mediating their electronic effects through the phenyl ring, promote the formation of the tetrahedral geometry, or may be a substituent capable of forming an intramolecular bond with the boron atom forcing the boronate into the tetrahedral conformation.

PBA derivatives are described having the general structure:

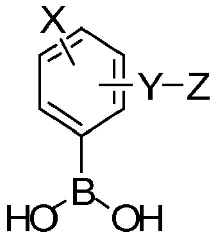

wherein X is an atom or group which, via electronic effect, promotes formation of tetrahedral geometry about the boron atom, Y is a linker, which may be an atom or group which, via electronic effect, promotes formation of tetrahedral geometry about the boron atom and Z is a polymerisable group. Specifically 2-acrylamido-phenylboronic acid and 3-acrylamido-phenylboronic acid were shown to provide significant response to glucose.

The known hydrogel technologies show a number of limitations for use in-vivo physiological conditions in blood analyte monitoring or regulation applications.

Another important problem encountered with hydrogels that contain proteins such as glucose oxidase or Con A is the leakage of proteins from the gel. Particularly Con A which is known to be a toxic compound. To reduce leakage, it has been proposed to covalently attach proteins to the polymer backbone (see for instance: Tanna S, Sahota T, Clark J, Taylor MJ: A covalently stabilised glucose responsive gel formulation with a Carbopol (R) carrier. Journal of Drug Targeting 2002, 10, 411-418. Tanna S, Sahota T, Clark J, Taylor MJ: Covalent coupling of concanavalin A to a Carbopol 934P and 941 P carrier in glucose-sensitive gels for delivery of insulin. Journal of Pharmacy and Pharmacology 2002, 54,1461-1469). However, the covalent attachment of proteins requires some synthetic efforts and may induce modifications of the biological functions of the proteins.

One major constraint to build a sensor for *in-vivo* applications is that all the components have to be sterilized. Hydrogels that contain proteins cannot be easily sterilized. Indeed, proteins such as glucose oxidase or Con A are sensitive to heat, which means that they cannot be autoclaved, and are denatured by gamma radiations.

An additional constraint for *in-vivo* sensor applications is that all the components used in the analyte responsive hydrogel should be biocompatible in order to prevent inflammation (acute and chronic) and fibrous encapsulation of the sensor which leads to a loss of sensibility of the sensor. Indeed, the coating itself can also lead to an undesirable response (Beckert WH, Sharkey MM: Mitogenic Activity of Jack Bean (Canavalia-Ensiformis) with Rabbit Peripheral Blood Lymphocytes. International Archives of Allergy and Applied Immunology 1970, 39, 337).

Further, for clinical applications, and especially for closed loop systems in diabetes treatment as described above, ever changing glucose concentrations demand hydrogels that can switch reproducibly and with rapid response onset times on a long-term basis. However, the response of bulk hydrogels upon changes in the environmental glucose concentration occurs too slowly, ranging from tens of minutes to hours.

The volume change process of gels is generally determined by the cooperative diffusion of the polymer in the solvent. As a result, swelling and shrinking of gels is quite slow because the diffusion coefficient of polymers is on the order of $10^{-7}$ $cm^2/s$, while that of water and small ions is on the order of $10^{-5}$ $cm^2/s$. (Katoa N, Gehrke SH: Microporous, fast response cellulose ether hydrogel prepared by freeze-drying, Colloids and Surfaces B: Biointerfaces 2004, 38, 191-196).

The sorption/desorption of solvents by gels is often described by a simple diffusion-controlled process; the polymer network motion of the conventional, non-porous gel is controlled by a diffusional process of polymers and the solvents. Fick's law of diffusion can be applied to the sorption/desorption process of gels and an apparent polymer solvent diffusion coefficient Dp in a polymer-fixed frame of reference is obtained by fitting the following equation to the macroscopic swelling/shrinking data in the case of the flat gel sheet:

$$\frac{M_t}{M_\infty} = 1 - \sum_{n=0}^{\infty} \left[ \frac{8}{(2n+1)^2 \pi^2} \right] \exp \left[ -(2n+1)^2 \pi^2 \frac{\mathrm{D_p} t}{L^2} \right]$$

where L is the initial thickness of the flat gel sheet.

The response time of such gels to the environmental change can be reduced by decreasing the characteristic diffusion path length i.e. decreasing L. (Katoa N, Gehrke SH: Microporous, fast response cellulose ether hydrogel prepared by freeze-drying, Colloids and Surfaces B: Biointerfaces 2004, 38, 191-196).

Reducing the hydrogel dimensions may be one potential way of shortening the response time. However reduction of the hydrogel dimensions tends to reduce the hydrogel integrity and may induce modifications in the response of the hydrogel to external stimuli (e.g. reduced swelling, reduced reactivity) which have negative impact on the utility of the hydrogel. For clinical applications, and especially for closed loop systems in diabetes treatment as described above, it is of utmost importance that the integrity, and response characteristics, of the hydrogel be maintained under clinical conditions.

Some systems have been proposed with the aim of maintaining hydrogel integrity. For instance the clamping of the hydrogel polymer in-between dialysis membranes (Kim JJ, Park K, Modulated insulin delivery from glucose-sensitive hydrogel dosage forms, Journal of Controlled Release 2001, 77, 39-47), however this results in further increasing the overall response time. It has also been proposed to cast the hydrogel around a mechanical support (M. Tang RZ, A. Bowyer, R. Eisenthal, J. Hubble,: A reversible hydrogel membrane for controlling the delivery of macromolecules, Biotechnology and Bioengineering 2003, 82, 47-53) or hydrogel nanoparticles within a polymer matrix. The polymer casting route does not allow for very thin hydrogel membranes to be fabricated, however, and slow response times will always result. Micro-fluidic concepts have also been investigated whereby hydrogel expansion/contraction depending on glucose concentration provides mechanical opening/closing of a microvalve gate, but with the same limitations (see for instance: Eddington DT, Beebe DJ, A valved responsive hydrogel microdispensing device with integrated pressure source. Journal of Microelectromechanical Systems 2004, 13, 586-593).

An object of the invention is to provide an analyte responsive membrane for use in a medical device for the measurement or regulation of analyte levels in a patient which overcomes some or all of the above-described limitations of known analyte responsive hydrogel membranes.

It would be advantageous to provide an analyte responsive membrane which has good membrane integrity, for application in a medical device for the measurement or regulation of analyte levels.

It would be advantageous to provide an analyte responsive membrane which provides rapid and selective response to changes in analyte concentration.

It would be advantageous to provide an analyte responsive membrane which provides reversible and reproducible reactivity to changes in analyte concentration on a long-term basis.

Objects of this invention have been achieved by providing a glucose responsive membrane according to claim 1, and by providing a method for the manufacture of a glucose responsive membrane according to claim 9, and by providing a medical device for the monitoring or regulation of glucose concentration according to claim 13.

Disclosed herein is a glucose responsive membrane, for use in a medical device for the monitoring and/or regulation of blood glucose levels in a patient, comprising a nanoporous support substrate and a glucose responsive hydrogel coating on the nanoporous support substrate. The glucose responsive hydrogel is strongly attached to a surface of the nanoporous support through covalent bonds or electrostatic interactions. It is preferred that the hydrogel is covalently attached to a surface of the nanoporous substrate.

The glucose responsive hydrogel advantageously comprises phenylboronic acid functional groups, and reversibly changes its three-dimensional configuration and/or surface properties in response to changes in glucose concentration occurring in the medium contacting the hydrogel under physiological conditions.

Dynamic flow properties through the membrane are controlled by the glucose responsive hydrogel coating.

The glucose responsive membrane of the present invention reversibly changes its hydraulic permeability in response to glucose concentration, making it possible to control hydraulic flow rate through the membrane.

The support substrate may be any suitable nanoporous substrate. Preferred substrates include nanoporous polypropylene, polyethylene, cellulose or alumina.

The hydrogel is preferably formed at the surface of the nanoporous substrate by a controlled surface-initiated polymerisation technique, by which advantageously the thickness of the hydrogel layer and its composition may be closely controlled.

Advantageously the composite membrane of the present invention has good hydrogel integrity and long-term stability, whilst providing a rapid response time to changes in glucose concentration.

The glucose responsive membrane of the present invention advantageously provides significant, selective and reversible response to changes in glucose concentration.

In a preferred embodiment the hydrogel is formed by a controlled surface initiated radical polymerisation process, preferably by surface-initiated atom transfer radical polymerisation (SI-ATRP), from an initiator group covalently attached to the substrate surface.

Advantageously the use of a controlled surface initiated polymerisation process for the formation of the hydrogel coating from the nanoporous substrate surface allows the grafting density, i.e. the distance between grafted polymer chains, as well as the thickness and composition of the hydrogel layer to be accurately controlled. Specifically a thin

layer of the hydrogel may be obtained on the nanoporous support substrate with very good hydrogel integrity and stability properties.

Advantageously the hydrogel is attached to a least part of the surface of the internal walls of the pores of the nanoporous substrate, thereby at least partially coating the internal walls of at least a part of the pores of the nanoporous substrate.

The glucose responsive membrane advantageously may further comprise non-fouling functional groups to prevent the non-specific adhesion of proteins, present in interstitial fluid, to the surface of the glucose responsive membrane. In a particular embodiment of the invention a layer of bio-compatible polymer is bound to the glucose responsive hydrogel layer. Advantageously a thin layer of biocompatible polymer may be covalently attached to the glucose responsive hydrogel by a controlled surface initiated polymerisation process.

Also provided herein is a method of preparation of a glucose responsive membrane for use in medical device for the monitoring or regulation of blood glucose levels in a patient, according to claim 16.

There is now provided a medical device for the monitoring and/or regulation of glucose levels in a patient including a glucose responsive membrane, which reversibly changes its hydraulic permeability subject to changes in glucose concentration, said membrane comprising a nanoporous support substrate and a glucose responsive hydrogel coating covalently attached to a surface of the nanoporous support substrate. The glucose responsive hydrogel advantageously comprises a polymeric matrix functionalised with phenylboronic acid moieties. Said medical device may optionally include means for administration of a quantity of a drug capable of adjusting glucose concentration, according to a determined glucose concentration.

Advantageously a medical device for the monitoring or regulation of glucose levels is based on mechanical sensing methods. According to a preferred embodiment, the medical device determines glucose concentration in a patient body fluid based on measurement of a flow resistance of a liquid through the glucose responsive membrane.

Further objects, advantageous features and aspects of the invention will be apparent from the claims and the following detailed description and examples, in conjunction with the figures in which:

Figure 1 shows a schematic illustration of selected variants of processes for the preparation of a glucose responsive membrane according to different embodiments of the present invention.

Figure 2 shows a schematic illustration of part of a device for monitoring or regulating glucose levels comprising a glucose responsive membrane according to an embodiment of the present invention.

The nanoporous support substrate for the membrane may consist of any suitable nanoporous material. Examples of commercially available nanoporous substrate materials include, for example metal oxides, silica, or polymeric substrates such as polyethylene, polypropylene, polyvinylidene difluoride (PVDF), polycarbonate, cellulose (regenerated cellulose, cellulose acetate, cellulose nitrate and cellulose ester), polyethersulfone (PES), nylon, Teflon® (PTFE). Particularly nanoporous alumina membrane substrates or nanoporous polymeric substrates such as nanoporous polypropylene substrates may be considered.

The pore size of the pores of the nanoporous substrate may generally vary between 2 and 800 nm, preferably pore size is not more than 400nm. At a substrate pore size of above 400 nm the control of hydraulic permeability properties of the membrane support substrate with the glucose responsive hydrogel coating layer tends to become less effective. A pore size of between 20nm and 300nm, e.g. between 50nm and 150nm, may be preferred.

A possible nanoporous substrate is nanoporous cellulose. Nanoporous cellulose membranes, e.g. re-generated cellulose, cellulose acetate or cellulose ester, are commercially available with pore sizes varying generally between 2nm and 1000nm. Porous hollow fibres made of cellulose, with pore size varying generally between 2nm and 15nm are also commercially available. Nanoporous cellulose substrates are potentially suitable for use in transcutaneous analyte sensor and regulation devices.

One type of preferred substrate materials are inert polymers such as polypropylene. Polyproylene and polyethylene substrates have found acceptance for a wide range of bio-medical applications. Compared to cellulosic materials polypropylene and polyethylene have a better durability, are more stable regarding hydrolysis, and are tolerant to aggressive chemicals which allows for a wide range of chemical modifications. Polypropylene and polyethylene nanoporous membranes with pore sizes varying generally between 20nm and 500nm are commercially available. Porous hollow fibres made of polypropylene and polyethylene are also commercially available, with pore size varying generally between 100nm and 300nm. Particularly nanoporous polypropylene hollow fibres are of interest for application in needle-type insertion members of a medical device.

Another preferred substrate material is nanoporous alumina, e.g. produced by an anodization process. Alumina substrates have found acceptance for a wide range of bio-medical applications. Advantageously such nanoporous alumina substrates present a high porosity and a relatively uniform pore structure having substantially straight cylindrical pores. Commercially available nanoporous alumina membranes produced by anodic oxidization processes have a pore diameter dependent on, among other parameters, the applied voltage, varying generally between 5 and 200 nm.

The glucose responsive hydrogel as described herein may encompass any suitable known glucose responsive hydrogel which exhibits a reversible change in configuration subject to glucose concentration in the surrounding medium. Suitable glucose responsive hydrogels include glucose responsive hydrogels having a specific affinity for glucose, which exhibit selectivity for glucose over other moieties present in physiological fluids, such as other sugars (e.g. fructose, galactose), which are sensitive to glucose at physiological conditions (temperature, ionic strength and pH values), and can respond reversibly to high and low glucose concentrations repeatably and reproducibly over many cycles (preferably over hundreds or even thousands of cycles).

The glucose responsive hydrogel should exhibit resistance to hydraulic pressure.

Advantageously the glucose responsive hydrogels according to the present invention are phenylboronic acid based hydrogels, such as described above.

Generally the hydrogels may include polymeric matrix of suitable monomer groups, such as methacrylate, acrylate, methacrylamide, acrylamide or vinylic monomer groups, functionalised with the glucose binding moiety, e.g. phenyl boronic acid moieties. The hydrogel may include cross-linker moieties, such as ethylene glycol dimethacrylate, poly(ethylene glycol) dimethacrylate, *N*,*N*'-methylenebisacrylamide, ethylene dimethacrylate, to promote hydrogel structural integrity, and/or polymer matrix density.

Particularly preferred are glucose responsive hydrogels based on phenylboronic acid derivatives. Glucose responsive hydrogels based on phenylboronic acid derivatives show good properties for resistance to flux of molecules such as water and insulin. Glucose responsive hydrogels have been developed which exhibit good selectivity for glucose, are sensitive to glucose under physiological conditions, show significant glucose response, and respond reversibly and reproducibly to high and low glucose concentrations.

Further, advantageously glucose responsive hydrogels based on phenylboronic acid or derivatives are highly stable and are resistant to heat, they can therefore be easily sterilized, e.g. by autoclave, or gamma radiation. A further advantage of the use of phenylboronic acid based hydrogels over glucose responsive hydrogels containing proteins such as glucose oxidase or lectins is that problems due to leakage of the of the proteins from the gel can be avoided.

The swelling and/or collapse of glucose responsive hydrogels based on phenyl boronic acid, or a derivative thereof, depends on the competitive binding of phenyl boronic acid moieties in the hydrogel matrix with free glucose, or on the change of solubility of the polymer in water (in the case of a temperature sensitive polymer matrix functionalised with PBA moieties), dependant on glucose concentration in the surrounding medium.

The phenylboronic acid moieties may be protected or unprotected. Particular examples of possible phenyl boronic acid based hydrogels include those described for instance in G. J. Worsley, G. A. Tourniaire, K. E. S. Medlock, F. K. Sartain, H. E. Harmer, M. Thatcher, A. M. Horgan, J. Pritchard Clinical Chem. 2007, 53, 1820-1826, Yang XP, Lee MC, Sartain F, Pan XH, Lowe CR: Designed boronate ligands for glucose-selective holographic sensors. Chemistry-a European Journal 2006, 12, 8491-8497, Kikuchi A, Suzuki K, Okabayashi O, Hoshino H, Kataoka K, Sakurai Y, Okano T: Glucose-sensing electrode coated with polymer complex gel containing phenylboronic acid. Analytical Chemistry 1996, 68:823-828, WO 2004/081624, and as discussed above. In the case of the use of monomers functionalized with protected phenylboronic acid moieties for the preparation of the glucose sensitive coating, a deprotection step is required.

Preferred phenylboronic acid moieties include the unprotected phenyl boronic acid derivatives of the structural formula (I):

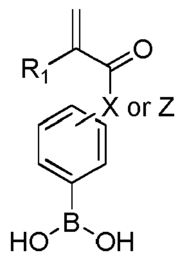

Formula (I),

wherein X = NH or O; $R_1$ = H or a $C_1$ to $C_4$ alkyl group; Z = a linker group. Preferably $R_1$ = H or a methyl group. The linker group Z may be any suitable linker group such as glycol or a $C_1$ to $C_{10}$ aliphatic chain or aromatic chain.

Preferred aliphatic chain groups include straight chain or branched $C_1$ to $C_{10}$ alkyl or $C_1$ to $C_{10}$ alkene groups. or formula (II):

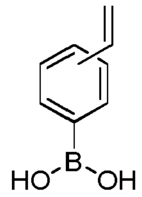

Formula (II),

and the protected pheylboronic acid derivatives of the structural formula (III):

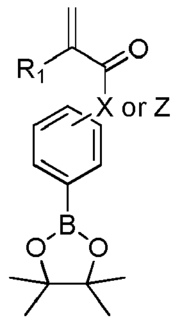

Formula (III),

wherein X, $R_1$ and Z are as defined above,
or (IV):

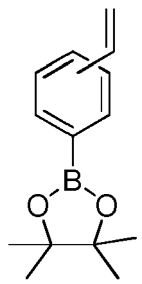

Formula (IV),

According to one embodiment preferred phenylboronic acid based hydrogels comprise a derivative of phenylboronic acid and a basic group such as a tertiary or quaternary amino group in the vicinity of the phenylboronic acid moieties, and/or a derivative phenylboronic acid modified with a tertiary or quaternary amino group.

Exemplary tertiary amino groups include a group of structural formula (V):

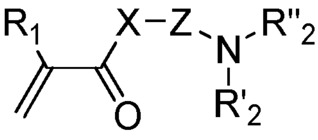

Formula (V),

wherein X, $R_1$ and Z are as defined above, and $R_2$' and $R_2$" are each individually a $C_1$ to $C_{10}$ alkyl group, preferably a $C_1$ to $C_4$ alkyl group. $R_2$' and $R_2$" on a same nitrogen group may be the same or different.

Exemplary quaternary amino groups include a group of structural formula (VI):

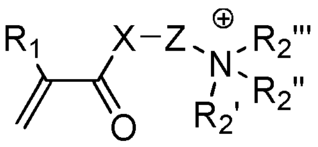

Formula (VI),

wherein X, $R_1$ and Z are as defined above, and $R_2$', $R_2$" and $R_2$"' are each individually a $C_1$ to $C_{10}$ alkyl group, preferably a $C_1$ to $C_4$ alkyl group. $R_2$' $R_2$" and $R_2$"' on a same nitrogen group may be the same or different.

Exemplary glucose responsive hydrogels are phenylboronic acid hydrogels comprising 3-(acrylamido)phenylboronic acid) or 2-(acrylamido)phenylboronic acid as the phenylboronic acid moiety. Particulary a phenylboronic acid hydrogel comprising 3-(acrylamido)phenylboronic acid) and a tertiary amine, such as (N-[3-(dimethylamino)propyl moiety), for instance as disclosed by G. J. Worsley, G. A. Tourniaire, K. E. S. Medlock, F. K. Sartain, H. E. Harmer, M. Thatcher, A. M. Horgan, J. Pritchard Clinical Chem. 2007, 53, 1820-1826; or a phenylboronic acid hydrogel comprising 2-(acrylamido)phenylboronic acid, for instance as disclosed by Yang XP, Lee MC, Sartain F, Pan XH, Lowe CR: Designed boronate ligands for glucose-selective holographic sensors. Chemistry-a European Journal 2006, 12:8491-8497, may be preferred.

For clinical applications, e.g. in a glucose sensor device, the glucose responsive membrane should be biocompatible in order to prevent inflammation (acute and chronic) and fibrous encapsulation of the membrane which leads to a loss of sensibility of the sensor. The hydrogel should be non-toxic and non-immunogenic. According to a preferred embodiment of the present invention the membrane further comprises anti-fouling groups. Examples of suitable bioinert groups for providing improved anti-biofouling properties include neutral groups such as polyethylene glycol (PEG), 2-hydroxyethyl and saccharide moieties, and charged groups such as phosphorylcholine moieties. PEG moieties are preferred due to the acceptance of PEG for pharmaceutical applications.

The anti-fouling groups may be introduced into the matrix of the glucose responsive hydrogel, for instance by copolymerisation of monomers functionalised with the anti-fouling groups. Advantageously the anti-fouling groups may be provided in a polymer layer attached covalently to the glucose responsive hydrogel layer, e.g. by a subsequent polymerisation process of monomers functionalised with anti-fouling groups, to form a block-copolymer.

Advantageously hydrogels according to the present invention show significant response to changes in glucose concentration, showing reversible and reproducible swelling properties subject to changes in glucose concentration. Hydrogels are able to provide good resistance to flow of water, and molecules such as insulin, due to their particular cross-linked matrix structure.

Advantageously the hydrogel is attached to a least part of the surface of the internal walls of the pores of the nanoporous substrate. An important advantage of the presence of the hydrogel on the pore walls is a synergy between the useful properties of the support substrate, such as stiffness, and those of the functional polymer layer.

The attachment of the polymers to the substrate surface may be achieved by "grafting to" techniques which involve the tethering of pre-formed functionalized polymer chains to a substrate under appropriate conditions, or "grafting from" techniques which involve covalently immobilizing an initiator species on the substrate surface, followed by a polymerization reaction to generate the polymer brushes. Creation of the polymer brushes by covalent attachment

methods advantageously provides good hydrogel integrity and long-term membrane stability properties.

For the synthesis of macromolecular layers via the "*grafting from*" route, conventional free radical polymerization initiated by electron beam irradiation, plasma treatment, or direct UV irradiation have been used extensively. An attractive alternative to conventional UV or plasma initiated polymerization is the use of surface-initiated controlled / "living" polymerization methods such as surface initiated atom transfer radical polymerisation (SI-ATRP), nitroxide-mediated processes (SI-NMP), reversible addition-fragmentation chain transfer polymerization (SI-RAFT), photo iniferter mediated polymerization (SI-PIMP) processes and ring-opening polymerisation (SI-ROP) processes. Advantages of these controlled/"living" surface initiated polymerisation processes include precise control over the thickness and composition of the resulting polymer film, and the ability to prepare block co-polymers by sequential activation of a dormant chain end in the presence of different monomers. As polymer chains start to grow from defined initiator sites at the surface, these techniques produce thin films in which polymer chains are tethered by their ends to the surface. These films are often referred to as polymer brushes.

In a preferred embodiment, the glucose responsive hydrogel is created at the substrate surface by a controlled/"living" surface initiated polymerisation (SIP) process. A surface initiated controlled / "living" polymerisation process of particular interest is surface-initiated atom transfer radical polymerisation (SI-ATRP), due to its robustness and synthetic flexibility. Further, water may be used as the main solvent used for most SI-ATRP processes, which is a particular advantage for application on an industrial scale. The use of a controlled/"living" surface initiated polymerisation process for the creation of the hydrogel layer on the nanoporous substrate surface advantageously enables precise control over the thickness, polymer chain density, and composition of the hydrogel layer.

For instance, surface initiated atom transfer radical polymerisation of monomers to form a polymer coating layer on the substrate surface can be carried out in accordance with known techniques, for example, such as described in a) Zhao, B.; Brittain, W. J. J. Am. Chem. Soc. 1999, 121, 3557-3558, b) Hussemann, M.; Malmstrom, E.; McNamara, M.; Mate, M.; Mecerreyes, D.; Benoit, D. G.; Hedrick, J. L.; Mansky, P.; Huang, E.; Russel, T. P.; Hawker, C. J. Macromolecules 1999, 32, 1424-1431, c) Matyjaszewski, K.; Miller, P. J.; Shukla, N.; Immaraporn, B.; Gelman, A.; Luokala, B. B.; Siclovan, T. M.; Kickelbick, G.; Vallant, T.; Hoffman, H.; Pakula, T. Macromolecules 1999, 32, 8716-8724, d) Ejaz, M.; Yamamoto, S.; Ohno, K.; Tsuji, Y.; Fukuda, T. Macromolecules 1998, 31, 5934-5926 for silicon wafers, such as described in von Werne, T.; Patten, T. E. J. Am. Chem. Soc. 1999, 121, 7409-7410 for silica, such as described in Sun, L.; Baker, G. L.; Bruening, M. L. Macromolecules 2005, 38, 2307-2314 for alumina, such as described in a) Shah, R. R.; Merreceyes, D.; Husemann, M.; Rees, I.; Abbott, N. L.; Hawker, C. J.; Hedrick, J. L. Macromolecules 2000, 33, 597-605, b) Kim, J.-B.; Bruening, M. L.; Baker, G. L. J. Am. Chem. Soc. 2000, 122, 7616-7617, c) Huang, W.; Kim, J.-B.; Bruening, M. L.; Baker, G. L. Macromolecules 2002, 35, 1175-1179 for gold, such as described in a) Fan, X.; Lin, L.; Dalsin, J. L.; Messersmith, P. B. J. Am. Chem. Soc. 2005, 127, 15843-15847, b) Zhang, F.; Xu, F. J.; Kang, E. T. Neoh, K. G. Ind. Eng. Chem. Res. 2006, 45, 3067-3073 for titanium oxide and such as described in Li, G.; Fan, J.; Jiang, R.; Gao, Y. Chem. Mater. 2004, 16, 1835-1837 for iron oxide, and as described in, for instance, US 6,949,292, US 6,946,164 and WO 98/01480.

In general, in controlled surface initiated polymerisation (SIP) processes, an initiator moiety is first chemically bound to the substrate surface, and then the polymerisation is carried out from the initiator moiety in the presence of an appropriate catalytic system. The initiating moiety generally consists of an anchoring group covalently attached to an initiating group, wherein the anchoring group is adapted to the material from which the support membrane is made of whereas the initiating group is adapted to the selected controlled SIP technique.

Dependent on the nature of the substrate material the substrate may exhibit chemical properties at its surface suitable for the binding of the initiator groups, e.g. having reactive groups, such as hydroxide groups, on their surface which act as the anchoring group for binding of the initiator group. Reactive groups may also be introduced onto the surface of the substrate by exposure to chemicals, coroner discharge, plasma treatment, etc. For example, piranha solution or plasma treatment can be used to hydroxylate, or activate, the surface of a silica or alumina substrate.

The initiator group is covalently bound to the substrate surface via the anchoring group at the substrate surface. The initiator group may be selected from known initiator groups. The choice of the initiator group for the controlled surface initiated polymerisation depends largely on the desired reaction conditions and the monomer(s) to be polymerised. Examples of suitable initiator species may be found, for example, in US 6,949,292, US 6,986,164, US 6,653,415 and US2006/0009550A1.

The initiator groups may be assembled onto the surface of the substrate in the presence of appropriate solvents.

Starting from the initiator moiety bound to the substrate surface a "living"/controlled surface initiated polymerisation is carried out with the monomers as desired for the formation of the polymer brushes. The "living"/controlled free radical polymerisation reaction is carried out in the presence of a suitable catalytic system. Typical catalytic systems comprise metal complexes containing transition metals e.g. copper, ruthenium or iron as the central metal atom. Exemplary metal catalysts include copper complexes such as copper chloride, copper bromides, copper oxides, copper iodides, copper acetates, copper perchlorate, etc.

In a particular embodiment, the glucose responsive hydrogel coating may be formed by SI-ATRP from a nanoporous alumina substrate. An initiator species, for instance as described in US 6,653,415, for example a bromoisobutyramido trimethoxysilane initiator group, or a chlorodimethylsilyl 2-bromo-2-methylpropanoate group, may be used. Other suitable initiator species include a cathecolic alkyl halide initiator group, such as 2-Bromo-N-[2-(3,4-dihydoxyphenyl)-ethyl]-propionamide, as described in US 2006/0009550.

In the case of a nanoporous cellulose substrate SIP may be carried out from the substrate surface using a suitable initiator capable of binding with hydroxyl groups on the cellulose substrate surface. For instance the cellulose hydroxyl groups may be esterified with 2-bromoisobutyryl bromide, or analogs thereof (Carlmark, A.; Malmström, E. J. Am. Chem. Soc. 2002, 124, 900-901). To enhance the accessibility of the hydroxyl groups and to facilitate higher degree of substitution, cellulose fibers may, for example, be pretreated with aqueous NaOH. After extensive washing with ethanol and tetrahydrofuran (THF), these substrates may, for example, be reacted with 2-chloro-2-phenylacetyl chloride and subsequently treated with phenyl magnesium chloride in the presence of carbon disulfide to generate a cellulose-bound RAFT agent (Roy, D.; Knapp, J. S.; Guthrie, J. T.; Perrier, S. Biomacromolecules 2008, 9, 91-99)

Polymeric substrates such as polyethylene (PE) or polypropylene (PP) that lack functional groups, which can act as handles to introduce functional groups that initiate or mediate SI-CRP, generally require a pretreatment or activation step. A variety of plasma and oxidative surface treatments are known for modifying inert polymer substrates with hydroxyl or carboxylic acid groups, which can then be further modified with initiator species, such as 2-bromoisobutyryl bromide or analogues, to allow SI-ATRP. For instance, ATRP initiating groups have been introduced onto the surface of polypropylene hollow fiber membranes using ozone pretreatment (Yao, F.; Fu, G.-D.; Zhao, J. P.; Kang, E.-T.; Neoh, K. G. J. Membr. Sci. 2008, 319, 149-157) and onto the surface of high-density polyethylene (HDPE) film using maleic anhydride activation (Yamamoto, K.; Miwa, Y.; Tanaka, H.; Sakaguchi, M.; Shimada, S. J. Polym. Sci., Part A: Polym. Chem. 2002, 40, 3350-3359).

Alternatively several protocols have been developed that allow the modification of "inert" polymer substrates with SI-CRP active functional groups in a single step. For example, polypropylene can be photobrominated to generate alkyl bromide groups that can be used directly to initiate SI-ATRP (Desai, S. M.; Solanky, S. S.; Mandale, A. B.; Rathore, K.; Singh, R. P. Polymer 2003, 44, 7645-7649). Another approach that allows the one step modification of "inert" polymer substrates is based on benzophenone photochemistry. Under UV radiation, benzophenone can abstract a hydrogen atom from neighboring aliphatic C-H groups to form a C-C bond. For example, the benzophenone group in benzophenonyl 2-bromoisobutyrate may be used as an anchor to promote the immobilization of the ATRP initiator group on polypropylene (Huang, J. Y.; Murata, H.; Koepsel, R. R.; Russell, A. J.; Matyjaszewski, K. Biomacromolecules 2007, 8, 1396-1399). Alternatively, benzophenone may be grafted onto high-density polyethylene (HDPE) and used as an initiator for reverse ATRP (Desai, S. M.; Solanky, S. S.; Mandale, A. B.; Rathore, K.; Singh, R. P. Polymer 2003, 44, 7645-7649.

The methods described above can suitably be used to prepare polypropylene and polyethylene substrates with functional groups that can initiate or mediate SI-CRP, e.g. SI-ATRP. Alternatively, polymer brushes can be grown from polymeric substrates, such as polypropylene or polyethylene, in the absence of such functional groups when the polymeric substrate is exposed to UV or γ-irradiation or is plasma treated. For example γ-irradiation may be used to initiate polymerization and cumyl phenyldithioacetate used as a RAFT agent for the grafting of polymer brushes from polypropylene substrates (Barner, L.; Perera, S.; Sandanayake, S.; Davis, T. P. J. Polym. Sci., Part A: Polym. Chem. 2006, 44, 857-864). Similarly, 1-phenylethyl phenyldithioacetate has been used as RAFT agent for the modification of the surface of polyethylene-co-polypropylene (PE-co-PP) sheets (Kiani, K.; Hill, D. J. T.; Rasoul, F.; Whittaker, M.; Rintoul, L. J. Polym. Sci., Part A: Polym. Chem. 2007, 45, 1074-1083). UV and γ-radiation have also been used to activate polyethylene substrates and allow reverse SI-ATRP (Yamamoto, K.; Tanaka, H.; Sakaguchi, M.; Shimada, S. Polymer 2003, 44, 7661-7669).

By the use of controlled surface initiated polymerisation techniques to create the glucose responsive hydrogel layer on the nanoporous support substrate, a thin layer of the hydrogel can be produced on the surface of the nanoporous support substrate and the thickness of the hydrogel layer can be precisely controlled. The thickness of the hydrogel layer produced in a specific SIP polymerisation reaction is controlled by the kinetics of the reaction which can be controlled in particular by controlling the length of time of the polymerisation reaction, or the concentration of monomers/catalyst.

The thickness of the hydrogel coating on the nanoporous substrate required to provide the desired flow rate properties for the coated membrane will depend, amongst other things, on the pore size and structure of the nanoporous substrate, and the structure and nature of the selected hydrogel, e.g. swelling properties of the selected hydrogel. As an example, a coating thickness of between 1nm and 200nm, for instance between 1 nm and 100nm, e.g. 5nm to 20nm, may be envisaged.

Advantageously the use of controlled surface initiated polymerisation techniques to form a glucose responsive hydrogel coating on the surface enables the preparation of a thin layer of the hydrogel which is strongly attached through covalent bonds to the surface of the nanoporous support substrate; thereby providing a glucose responsive membrane exhibiting a rapid response to changes in glucose concentration, whilst at the same time showing good hydrogel integrity and long term stability properties (e.g. over 5 to 7 days under pharmacological conditions) required for clinical applications.

A glucose responsive membrane according to the present invention may suitably be prepared by a process

comprising the steps of covalently binding an initiator group to a surface of a nanoporous support substrate, and subsequently forming a glucose responsive hydrogel at the surface of the nanoporous substrate via a controlled surface initiated polymerisation process from the initiator group.

According to an embodiment of the present invention the formation of the glucose responsive hydrogel at the surface of the nanoporous substrate via a controlled surface initiated polymerisation process may involve the co-polymerisation of monomers selected from methacrylate, acrylate, methacrylamide, acrylamide or vinylic monomers functionalised with glucose binding functional groups, methacrylate, acrylate, methacrylamide, acrylamide or vinylic monomers functionalised with tertiary or quaternary amino groups, methacrylate, acrylate, methacrylamide, acrylamide or vinylic monomers functionalised with active ester groups, cross-linker groups selected from di(methacrylate), di(acrylate), di (methacrylamide), di(acrylamide) or di(vinylic) monomer groups, methacrylate, acrylate, methacrylamide, acrylamide or vinylic monomers functionalised with 2-hydroxyethyl, polyethylene glycol or phosphorylcholine groups, non-functionalised methacrylate, acrylate, methacrylamide, acrylamide or vinylic monomers.

Phenylboronic acid, or derivatives of phenylboronic acid, glucose binding functional groups may be introduced in the glucose responsive hydrogel by direct co-polymerisation of monomers comprising glucose binding functional groups in a controlled surface initiated polymerisation process, from the initiator group.

Alternatively, glucose binding functional groups may be introduced in the glucose responsive hydrogel in a subsequent step by substitution of glucose binding functional group containing moieties at activated sites in the formed hydrogel.

Advantageously the glucose responsive hydrogel comprises anti-fouling functional groups to prevent the non-specific adhesion of proteins, present in interstitial fluid, to the surface of the glucose responsive membrane.

The anti-fouling groups, such as 2-hydroethyl or polyethylene glycol (PEG), may be introduced into the matrix of the glucose responsive hydrogel, for instance by copolymerisation of monomers functionalised with the anti-fouling groups in a controlled surface initiated polymerisation process from the initiator groups bound to the substrate surface.

Advantageously the anti-fouling groups may be provided in a polymer layer attached covalently to the glucose responsive hydrogel layer, e.g. by a subsequent controlled surface initiated polymerisation step of monomers functionalised with anti-fouling groups, to form a block-copolymer. The use of a controlled surface initiated polymerisation process enables the formation of a thin layer of biocompatible polymer covalently attached to the glucose responsive hydrogel, and of which the layer thickness can be precisely controlled. The protection of the glucose responsive layer with a thin layer of covalently attached biocompatible polymer brushes, to form a block co-polymer, in this way is particularly preferred in the case of a glucose responsive hydrogel based on phenylboronic acid moieties since glycoproteins such as $\gamma$-globulin, present at non negligible concentrations in interstitial fluid, are known to interact with phenylboronic acid moieties which could lead to undesirable foreign body reaction.

Examples of suitable process strategies for the preparation of a glucose sensitive hydrogel comprising phenylboronic acid moieties are illustrated schematically in Figure 1.

According to a first possible process strategy, illustrated in figure 1A, in a first controlled surface initiated polymerisation step from initiator groups (3) bound to the nanoporous support substrate (1) there are copolymerised

(i) methacrylate or acrylate or methacrylamide or acrylamide or vinylic monomers functionalized with unprotected phenylboronic acid moieties (5), preferably selected from unprotected phenylboronic acid moieties of formula (I) or (II), or protected phenyl boronic acid preferably selected from unprotected phenylboronic acid moieties of formula (I) or (II), and optionally,

(ii) monomers such as non-functionalized methacrylate or acrylate or methacrylamide or acrylamide or vinylic monomers, cross-linkers, and/or methacrylate or acrylate or methacrylamide or acrylamide or vinylic monomers functionalized with neutral tertiary amine groups (7), preferably selected from a group of formula (III), or charged quaternary amine groups, preferably selected from a group of formula (IV).

Optionally, methacrylate or acrylate or methacrylamide or acrylamide or vinylic monomers functionalized with anti-fouling functional groups, such as 2-hydroethyl or polyethylene glycol moieties, may be copolymerized with the above-listed monomers in the first polymerisation step or methacrylate or acrylate or methacrylamide or acrylamide or vinylic monomers functionalized with anti-fouling functional groups (9), such as 2-hydroethyl or polyethylene glycol moieties, may be polymerized in a second polymerization step to give a block-copolymer (Figure 1 D).

Suitable cross-linkers include groups of the general structural formula (VII):

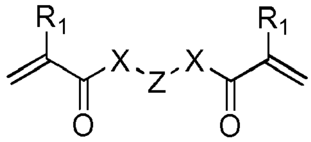

Formula (VII),

wherein X, $R_1$ and Z are as defined above. According to a second possible process strategy, illustrated in figure 1B, in a first controlled surface initiated polymerisation step from initiator groups bound to the nanoporous support substrate there are copolymerised

(i) methacrylate or acrylate or methacrylamide or acrylamide or vinylic monomers functionalized with active ester groups (11), and optionally,

(ii) monomers such as non-functionalized methacrylate or acrylate or methacrylamide or acrylamide or vinylic monomers and/or cross-linkers, such a cross-linker groups of formula (VII).

Optionally, monomers such as non-functionalized methacrylate or acrylate or methacrylamide or acrylamide or vinylic monomers monomers functionalized with anti-fouling functional groups, such as 2-hydroethyl or polyethylene glycol moieties, may be copolymerized with the above-listed monomers in the first polymerisation step or may be polymerized in a second polymerization step to give a block-copolymer (Figure 1 D).

In a subsequent step phenylboronic acid functional groups, and optionally tertiary or quaternary amino functional groups, are introduced at the active ester sites by nucleophilic substitution.

Suitable active ester functional groups include active ester groups of the structural formulae (VIII):

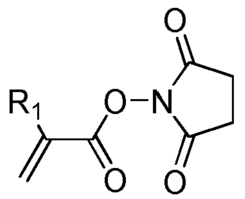

Formula (VIII),

or (IX)

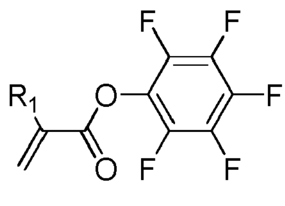

Formula (IX),

wherein $R_1$ is as defined above.

Phenylboronic acid functional groups may, for instance, be introduced at the active ester sites by nucleophilic substitution of a group of structural formula (X):

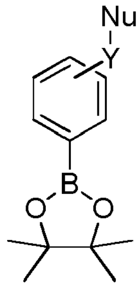

Formula (X),

or (XI):

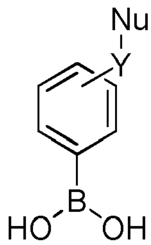

Formula (XI),

wherein Y = $NH_2$ or a linker group. The linker group may be any suitable linker group such as glycol or a $C_1$ to $C_{10}$ aliphatic chain or aromatic groups. Preferred aliphatic chain groups include straight chain or branched $C_1$ to $C_{10}$ alkyl or $C_1$ to $C_{10}$ alkene groups. Nu = a nucleophillic group. Suitable nucleophillic groups include $NH_2$ or OH.

Amino functional groups may, for instance, be introduced at the active ester sites by nucleophilic substitution of a group of structural formula (XII):

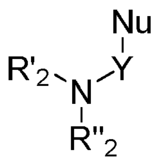

Formula (XII),

wherein Y, Nu, $R_2$' and $R_2$" are as defined above.

According to a third possible process strategy, illustrated in figure 1C, in a first controlled surface initiated polymerisation step from initiator groups bound to the nanoporous support substrate there are copolymerised

(iii) methacrylate or acrylate or methacrylamide or acrylamide or vinylic monomers functionalized with anti-fouling functional groups (9), such as 2-hydroethyl or polyethylene glycol moieties, and optionally,

(iv) monomers such as non-functionalized methacrylate or acrylate or methacrylamide or acrylamide or vinylic monomers and/or cross-linkers, such a cross-linker groups of formula (VII).

In a subsequent step anti-fouling functional groups (9), e.g. 2-hydroethyl or polyethylene glycol moieties, are modified with active esters (13), such as active ester groups of formula (VIII) or (IX).
In a subsequent step phenylboronic acid functional groups, and optionally tertiary or quaternary amino functional groups, are introduced at the active ester sites by nucleophilic substitution. Phenylboronic acid functional groups may, for instance, be introduced at the active ester sites by nucleophilic substitution of groups of structural formula (X) or (XI). Amino functional groups may, for instance, be introduced at the active ester sites by nucleophilic substitution of groups of structural formula (XII).

The strategies A and B can be combined. For example, in a first controlled surface initiated polymerisation step from initiator groups bound to the nanoporous support substrate there may be copolymerised

(i) methacrylate or acrylate or methacrylamide or acrylamide or vinylic monomers functionalized with neutral tertiary amine groups, preferably selected from a group of formula (III), or charged quaternary amine groups, preferably selected from a group of formula (IV),
(ii) methacrylate or acrylate or methacrylamide or acrylamide or vinylic monomers functionalized with active ester groups, such as active ester groups according to formula (VIII) or (IX), and optionally
(iii) non-functionalized methacrylate or acrylate or methacrylamide or acrylamide or vinylic monomers, cross-linkers, such a cross-linker groups of formula (VII).

Optionally, methacrylate or acrylate or methacrylamide or acrylamide or vinylic monomers functionalized with anti-fouling functional groups, such as 2-hydroethyl or polyethylene glycol moieties, may be copolymerized with the above-listed monomers in the first polymerisation step or may be polymerized in a second polymerization step to give a block-copolymer (Figure 1 D).

In a subsequent step phenylboronic acid functional groups, are introduced at the active ester sites by nucleophilic substitution. The phenylboronic acid functional groups may, for instance, be introduced at the active ester sites by nucleophilic substitution of groups of structural formula (X) or (XI).

The glucose responsive hydrogel coating layer formed according to the present invention has the effect of controlling the hydraulic flow properties of the nanoporous support substrate.

As discussed above the degree of swelling and/or collapse of hydrogel polymer matrices functionalised with phenyl boronic acid, or a derivative thereof, depends on glucose concentration in the surrounding medium. In the glucose responsive hydrogel coated membrane the degree of opening of the pores of the nanoporous support substrate, that is to say the size of open flow channel through the pores, is controlled by changes in the degree of swelling of the glucose responsive hydrogel, subject to changes in glucose concentration in the surrounding medium

Another important property of phenylboronic acid compounds in an aqueous medium is that they are in equilibrium between an uncharged and a charged form. Only charged phenylborates can form stable complex with glucose. Increasing glucose concentration increases the charged phenylborates, thus, enhancing the hydrophilicity of amphiphilic polymers having pendant phenylborate moieties (see J. Xingju, Z. Xinge, W. Zhongming, T. Dayong, Z. Xuejiao, W. Yanxia, W. Zhen, L. Chaoxing Biomacromolecules 2009, 10, 1337-1345).

It is considered by the inventors that the variation of hydrophilicity of the hydrogel in response to glucose concentration could influence the surface energy of the material coated with PBA-based hydrogel. Indeed, it has been demonstrated that the surface energy of capillaries in microfluidic systems plays a crucial role in flow behavior ( see L. Ionov, N. Houbenov, A. Sidorenko, M. Stamm, S. Minko Adv. Funct. Mater. 2006, 16, 1153-1160. A. Constable, W. Brittain, Colloids and Surfaces a-Physicochemical and Engineering Aspects, 2007, 308, 123-128. in addition to volume changes. Without wishing to be bound by any particular theory it is considered by the inventors that variation of hydrophilicity of the hydrogel in response to glucose concentration also plays a role in the control of the hydraulic permeability of the membrane in response to glucose concentration.

Attachment of the glucose responsive hydrogel to inner walls of pores of the nanoporous substrate provides enhanced control of flux properties through the membrane, since the swelling of the hydrogel coating present in the pore produces a restriction of open pore diameter (i.e. flow channel size) along the length of the pore. In addition where hydrogel is present in pores of the porous substrate the change in hydrophobicity of the hydrogel in response to glucose concentration may play an enhanced role in the control of hydraulic permeability of the membrane. The presence of the glucose responsive hydrogel in the pores of the nanoporous substrate promotes effective closing of the pores by the hydrogel in a swollen state, due to increased resistance to hydraulic flow through the pore.

The glucose responsive membranes of the present invention are able to show a rapid response time to changes in glucose concentration, whilst providing good hydrogel integrity and stability properties.

Advantageously, glucose responsive membranes according to the present invention show good selectivity for glucose and show significant response to changes in glucose concentration at physiological conditions. Advantageously, glucose responsive membranes according to the present invention show reversible and reproducible swelling properties subject to changes in glucose concentration. Advantageously, glucose responsive membranes according to the present

invention show good resistance to flux of water, and molecules such as insulin.

Further, glucose responsive membranes of the present invention show good bio-compatibility properties.

The glucose responsive membranes of the present invention having a rapid response time to changes in glucose concentration, whilst having good hydrogel integrity and stability properties, make the membranes of the present invention particularly advantageous for the use in medical device applications for the monitoring or regulation of glucose levels.

Advantageously glucose responsive membranes according to the present invention comprising phenylboronic acid based glucose responsive hydrogel can be easily sterilised, e.g. by autoclave or gamma radiation, as required for in-vivo clinical applications.

Particularly, the coated membranes of the present invention are advantageously used in glucose sensor device, or a medical device for the treatment of patients with diabetes, particularly a closed loop system integrating glucose sensor and medication delivery.

Advantageously the medical device for the monitoring or regulation of glucose levels is based on mechanical sensing methods. According to a preferred embodiment, the medical device determines glucose concentration in a patient body fluid based on measurement of a flow resistance of a liquid through the glucose responsive membrane.

According to one embodiment of a medical device according to the present invention, as illustrated in figure 2 the glucose responsive membrane of the present invention, comprising a nanoporous support substrate (20), and a glucose responsive hydrogel coating layer (22) may form a bio-interface of part of a needle-like insertion member (24) to be inserted in a patient to contact with e.g. interstitial fluid, blood or tear fluid. In order to measure glucose concentration in the medium surrounding the needle-like insertion member a liquid flux (26) is produced in the insertion member, and the resistance to flux of this liquid through the glucose responsive membrane is measured. The change in volume and/or surface properties of the glucose response hydrogel layer attached to the support substrate, subject to the glucose concentration in the medium surrounding the needle-like insertion member, has the effect of decreasing or increasing the resistance to flux of the liquid through the membrane. The resistance to flux of the liquid through the membrane is measured using a flux sensor device, and this value used to determine glucose concentration in the medium surrounding the needle-like insertion member.

A medication capable of regulating blood glucose levels, e.g. insulin, may be delivered by the medical device in response to the determined glucose concentration.

According to a particular embodiment the glucose responsive hydrogel (22) may comprise phenyl boronic acid moieties (28) and tertiary amine moieties (30). At low glucose concentration in the surrounding medium the glucose responsive hydrogel has an expanded configuration (32), thereby closing or narrowing the pores of the nanoporous substrate, and the swollen hydrogel layer on the surface of the nanoporous substrate provides a high resistance to flux of the liquid through the membrane. At a high glucose concentration glucose (34) is bound by the phenylboronic acid moieties and contraction of the hydrogel occurs, whereby the contracted hydrogel layer (34) on the surface of the nanoporous substrate provides a low resistance to flux of the liquid through the membrane. Advantageously, the closing of the pores of the nanoporous membrane substrate at low glucose concentration in this way may increase the sensitivity of the system in the hypoglycaemic region, which is known to be difficult to monitor accurately with electrochemical sensors.

The medical device may, for example, have a construction of medical device for glucose monitoring and for drug delivery similar to that described in PCT/IB2008/054348, and wherein the medical device comprises an implantable member, having a needle-like form, for insertion into a patient, comprising a porous membrane, a pressure generating means adapted to deliver a liquid to the porous membrane, and a sensor adapted to measure flow resistance of the liquid through the membrane. A glucose sensitive membrane according to the present invention, which changes it hydraulic permeability subject to changes in glucose concentration in the medium contacting the membrane, may be used in the place of the porous membrane described in PCT/IB2008/054348.

Glucose concentration is measured by pumping a discrete volume of liquid towards the membrane, measuring a value correlated to a resistance against flow of the liquid through the membrane, and calculating a glucose concentration based on the measured value correlated to flow resistance through the porous membrane.

The liquid may comprise insulin, such that it is possible for the device to provide e.g. a basal rate of insulin through glucose responsive membrane. The medical device may comprise a separate channel for drug delivery, such that a bolus insulin dose may be administered through this separate channel as required, according to the determined glucose concentration.

Other constructions for the glucose sensor or medication delivery device may be envisaged.

## Claims

1. A glucose responsive membrane for use in a medical device for the monitoring or regulation of glucose levels

comprising;
a nanoporous support substrate, and
a layer of a glucose responsive hydrogel, attached through covalent bonds or electrostatic interactions to a surface of the nanoporous substrate, said glucose responsive hydrogel comprising a polymeric matrix containing phenyl boronic acid functional groups.

**2.** A membrane according to claim 1 wherein the hydrogel is attached to at least a part of the surface of the internal walls of pores of the nanoporous substrate.

**3.** A membrane according to claim 1 or 2 wherein the hydrogel is formed by a controlled surface-initiated polymerisation process from the nanoporous substrate.

**4.** A membrane according to any one of claims 1 to 3 wherein the glucose responsive hydrogel comprises 2-acrylamido-phenylboronic acid or 3-acrylamido-phenylboronic acid.

**5.** A membrane according to any one of claims 1 to 4 wherein the glucose responsive hydrogel further comprises a tertiary or quaternary amino group.

**6.** A membrane according to any one of claims 1 to 5 wherein the hydrogel comprises polymerised methacrylate, acrylate, methacrylamide, acrylamide or vinylic monomers.

**7.** A membrane according to any one of claims 1 to 6 wherein the support substrate is a nanoporous polypropylene, nanoporous polyethylene or nanoporous alumina substrate.

**8.** A membrane according to any one of claims 1 to 7 wherein the membrane comprises a layer of biocompatible polymer, comprising anti-fouling functional groups, attached through covalent bonds to the glucose responsive hydrogel coating layer.

**9.** A method for the preparation of a glucose responsive membrane comprising;
covalently binding an initiator group to a surface of a nanoporous support substrate,
and subsequently forming a layer glucose responsive hydrogel comprising phenylboronic acid functional groups at the surface of the nanoporous substrate via a controlled surface initiated polymerisation process from the initiator group.

**10.** A method according to claim 9 comprising a step of forming a layer of biocompatible polymer covalently attached to the glucose responsive hydrogel via a controlled surface initiated polymerisation process of monomer groups functionalised with anti-fouling moieties.

**11.** A method according to claim 9 or 10 for the preparation of a glucose responsive membrane comprising;
covalently binding an initiator group to a surface of a nanoporous support substrate,
and subsequently carrying out a polymerisation by a controlled surface initiated polymerisation process, from the initiator group, of monomers selected from methacrylate, acrylate, methacrylamide, acrylamide or vinylic monomers functionalised with phenylboronic acid functional groups; methacrylate, acrylate, methacrylamide, acrylamide or vinylic monomers functionalised with tertiary or quaternary amino groups; methacrylate, acrylate, methacrylamide, acrylamide or vinylic monomers functionalised with active ester groups; cross-linker groups; and,
optionally, monomers selected from acrylic or methacrylic monomers functionalised with 2-hydroethyl or polyethylene glycol moieties.

**12.** A method according to any one of claims 9 to 11 wherein phenylboronic acid functional groups are introduced in the glucose responsive hydrogel by direct co-polymerisation of monomers comprising phenylboronic acid functional groups by a controlled surface initiated polymerisation process, from the initiator group; or in a subsequent step by substitution of phenylboronic acid functional group containing moieties at activated sites in the formed hydrogel.

**13.** A medical device for the monitoring or regulation of glucose levels in a patient comprising;
an implantable member comprising a glucose responsive biointerface membrane, which reversibly changes its hydraulic permeability subject to changes in glucose concentration occurring in the medium surrounding the implantable member;
said membrane comprising a nanoporous support substrate,

and a layer of a glucose responsive hydrogel, attached through covalent bonds or electrostatic interactions to a surface of the nanoporous substrate, said glucose responsive hydrogel comprising a polymeric matrix functionalised with phenylboronic acid functional groups,.

**14.** A medical device according to claim 13 comprising a pressure generating means configured to deliver a liquid to the glucose responsive membrane; and a sensor adapted to measure a flow resistance of said liquid through the glucose responsive membrane; whereby glucose concentration is determined based on the flow resistance of the liquid through the glucose responsive membrane.

**15.** Use of a glucose responsive membrane according to any one of claims 1 to 8 as a bio-interface membrane in a medical device for the monitoring or regulating of blood glucose levels in a patient.

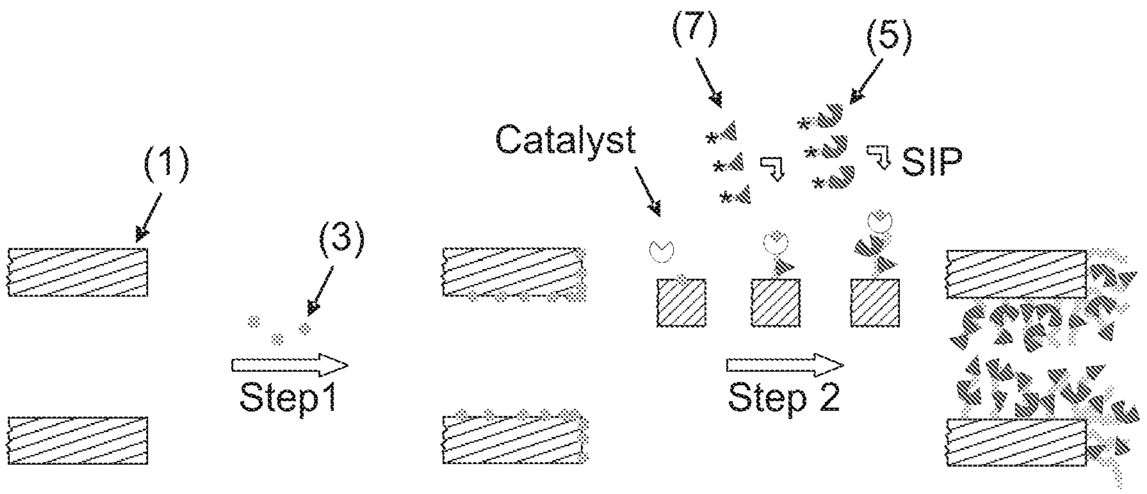


**Figure 1A**

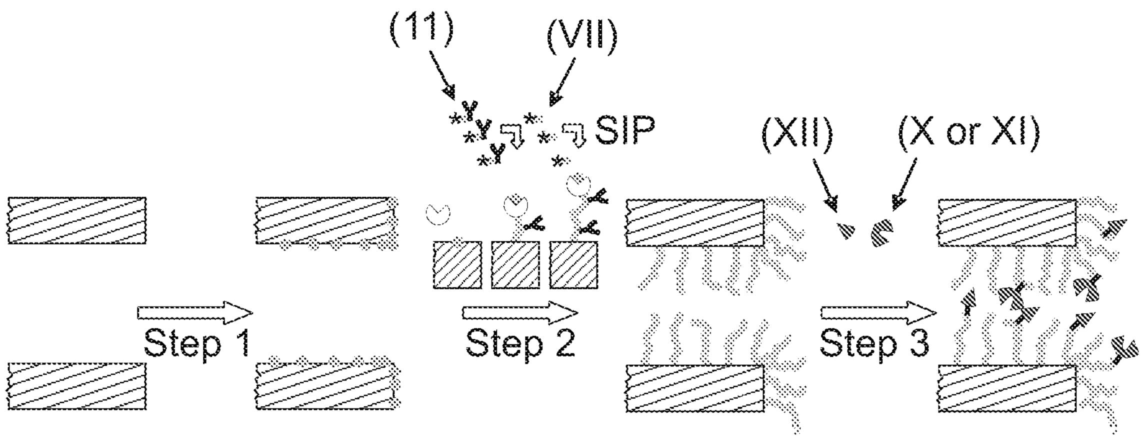


**Figure 1B**

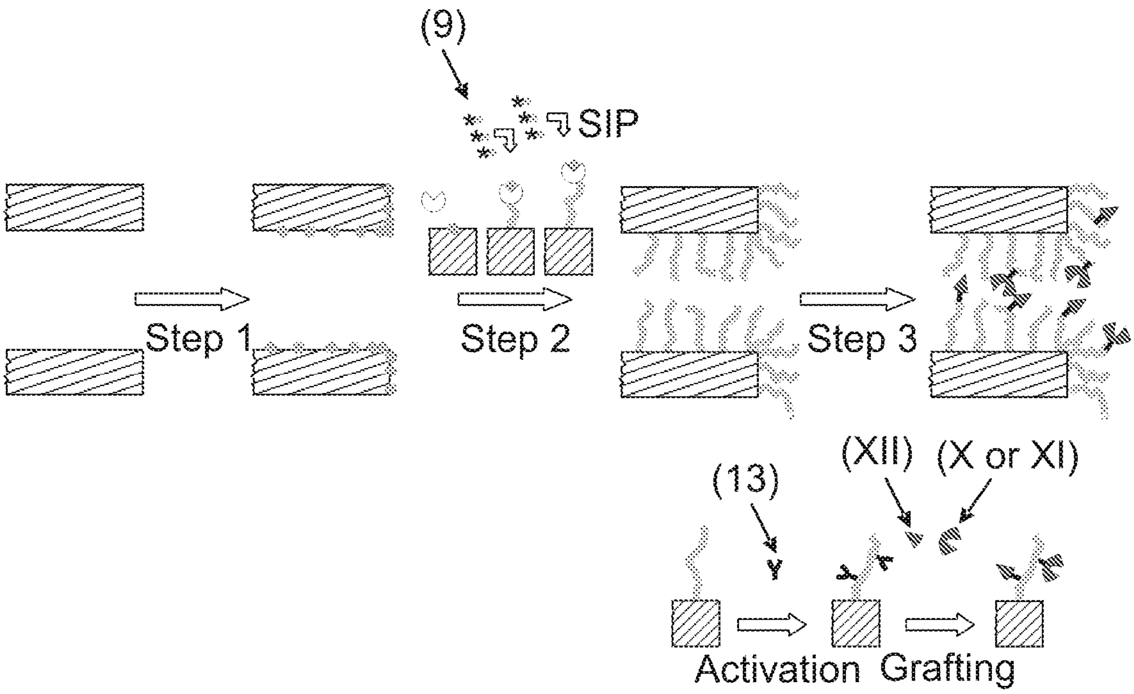


**Figure 1C**

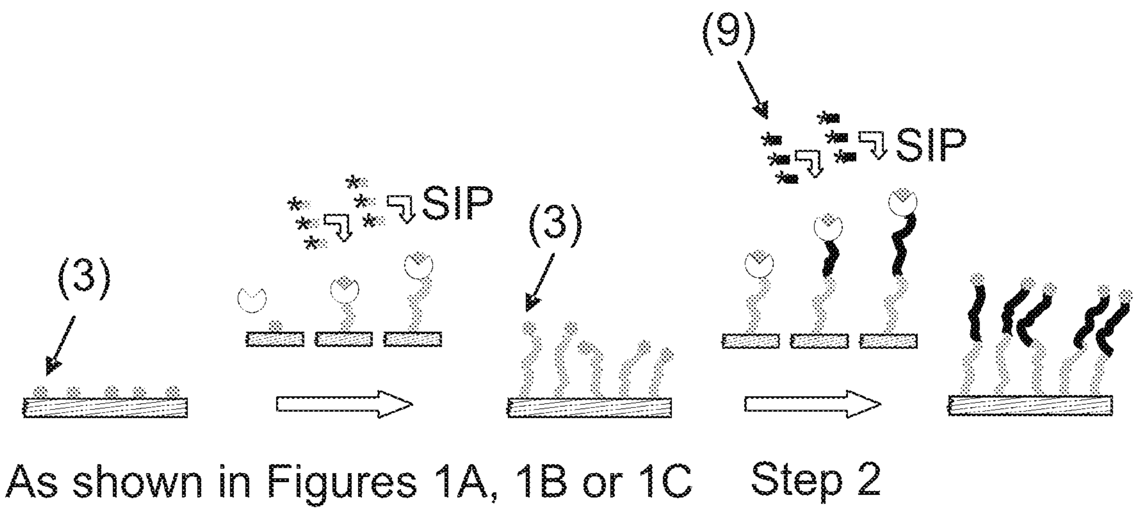


**Figure 1D**

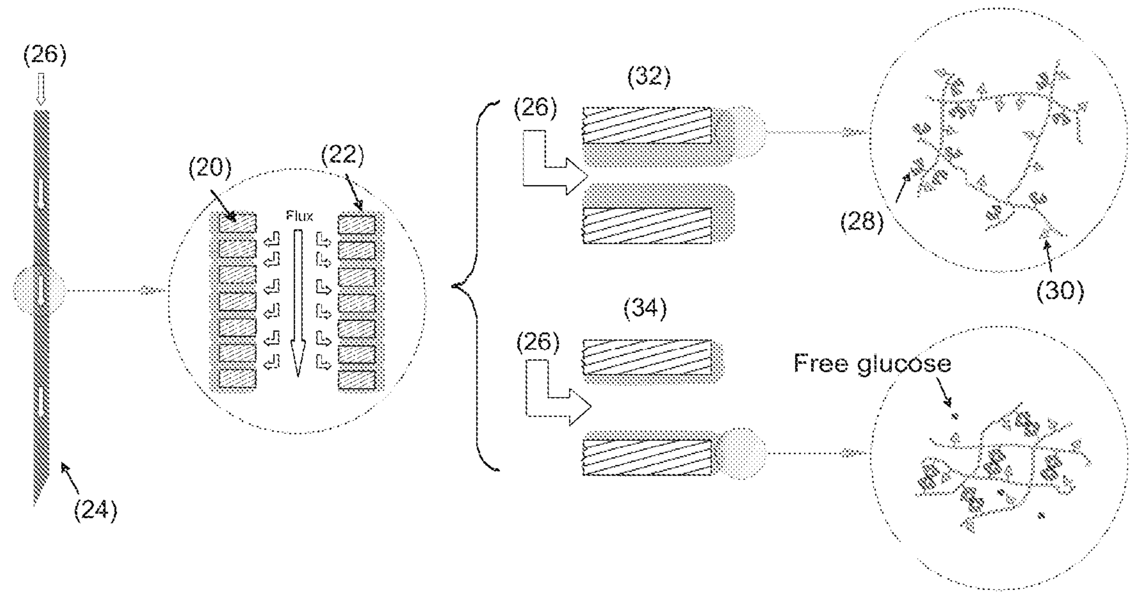


Figure 2

Europäisches Patentamt
European Patent Office
Office européen des brevets

# EUROPEAN SEARCH REPORT

Application Number
EP 09 16 7572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br>Y | LEI MING ET AL: "A hydrogel-based implantable micromachined transponder for wireless glucose measurement" DIABETES TECHNOLOGY AND THERAPEUTICS, MARY ANN LIEBERT, LARCHMONT, NY, US, vol. 8, no. 1, 1 February 2006 (2006-02-01), pages 112-122, XP002499731 ISSN: 1520-9156<br>* the whole document * | 1-12<br><br><br><br><br><br><br><br><br>13-15 | INV.<br>C12Q1/00 |
| Y | WO 2009/053911 A (SENSILE PAT AG [CH]; STRAESSLER SIGFRID [CH]; BEYER UWE [CH]) 30 April 2009 (2009-04-30)<br>* page 17, paragraph 1 *<br>* claims 1,11 * | 13-15 | |
| Y | US 2002/155425 A1 (HAN IN SUK [US] ET AL) 24 October 2002 (2002-10-24)<br>* paragraphs [0053], [0084] * | 13-15 | |
| Y | BALDI A ET AL: "A hydrogel-actuated smart microvalve with a porous diffusion barrier back-plate for active flow control" PROCEEDINGS OF THE IEEE 15TH. ANNUAL INTERNATIONAL CONFERENCE ON MICRO ELECTRO MECHANICAL SYSTEMS. MEMS 2002. LAS VEGAS, NV, JAN. 20 - 24, 2002; [IEEE INTERNATIONAL MICRO ELECTRO MECHANICAL SYSTEMS CONFERENCE], NEW YORK, NY : IEEE, US, vol. CONF. 15, 1 January 2002 (2002-01-01), pages 105-108, XP010577606 ISBN: 978-0-7803-7185-9<br>* figure 2 *<br>-/-- | 13-15 | TECHNICAL FIELDS SEARCHED (IPC)<br>C12Q |

The present search report has been drawn up for all claims

| Place of search | Date of completion of the search | Examiner |
|---|---|---|
| The Hague | 12 January 2010 | Gundlach, Björn |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 16 7572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GU Y ET AL.: "Modulation of drug delivery rate by hydrogel incorporating mems devices modulation of drug delivery rate by hydrogel incorporating mems devices" PROCEEDINGS, IEEE MICRO ELECTRO MECHANICAL SYSTEMS, 2 May 2002 (2002-05-02), - 4 May 2002 (2002-05-04) pages 406-409, XP008117066<br>* the whole document *<br>----- | 1-7, 9-13,15 | |

TECHNICAL FIELDS SEARCHED (IPC)

The present search report has been drawn up for all claims

2

| Place of search | Date of completion of the search | Examiner |
|---|---|---|
| The Hague | 12 January 2010 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 16 7572

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2010

| Patent document cited in search report | Publication date | Patent family member(s) | Publication date |
|---|---|---|---|
| WO 2009053911 A | 30-04-2009 | EP 2052677 A1 | 29-04-2009 |
| US 2002155425 A1 | 24-10-2002 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060224141 A
- WO 03047426 A1
- WO 8901794 A
- WO 890174 A
- WO 2004081624 A
- US 6949292 B
- US 6946164 B
- WO 9801480 A
- US 6986164 B
- US 6653415 B
- US 20060009550 A1
- US 20060009550 A


### Non-patent literature cited in the description


- **T. Miyata ; T. Uragami ; K. Nakamae.** *Adv. Drug Deliver. Rev.,* 2002, vol. 54, 79
- **Y. Qiu ; K. Park.** *Adv. Drug. Deliver. Rev.,* 2001, vol. 53, 321
- **S. Chaterji ; I. K. Kwon ; K. park.** *Prog. Polym. Sci.,* 2007, vol. 32, 1083
- **N. A. Peppas.** *J. Drug Del. Sci. Tech.,* 2004, vol. 14, 247-256
- **G. Albin ; T.A. Horbett ; B.D. Ratner.** *J. Controlled Release,* 1985, vol. 2, 153
- **K. Ishihara ; M. Kobayashi ; I. Shinohara.** *Polymer J.,* 1984, vol. 16, 625
- **Y. Ito ; M. Casolaro ; K. Kono ; I. Yukio.** *J. Controlled Release,* 1989, vol. 10, 195
- **M. J. Taylor ; S. Tanna.** *J. Pharm. Pharmacol.,* 1994, vol. 46, 1051
- **M. J. Taylor ; S. Tanna ; P. M. Taylor ; G. Adams.** *J. Drug Target.,* 1995, vol. 3, 209
- **S. Tanna ; M. J. Taylor.** *J. Pharm. Pharmacol.,* 1997, vol. 49, 76
- **S. Tanna ; M. J. Taylor.** *Pharm Pharmacol. Commun.,* 1998, vol. 4, 117
- **S. Tanna ; M. J. Taylor.** *Proc. Int. Symp. Contr. Rel. Bioact. Mater.,* 1998, vol. 25, 737B
- **S. Tanna ; M. J. Taylor ; G. Adams.** *J. Pharm. Pharmacol.,* 1999, vol. 51, 1093
- **K. Nakamae ; T. Miyata ; A. Jikihara ; A. S. Hoffman.** *J. Biomater. Sci. polym. Ed.,* 1994, vol. 6, 79
- **C. J. Ward ; P. Patel ; T. D. James.** *Org. Lett.,* 2002, vol. 4, 477
- **S. Kitano ; Y. Koyama ; K. Kataoka ; T. Okano ; Y. Sakurai.** *J. Controlled Release,* 1992, vol. 19, 161
- **K. Kataoka ; H. Miyazaki ; T. Okano ; Y. Sakurai.** *Macromolecules,* 1994, vol. 27, 1061
- **T. Aoki ; Y. Nagao ; K. Sanui ; N. Ogata ; A. Kikuchi ; Y. Sakurai ; K. Kataoka ; T. Okano.** *Polym. J.,* 1996, vol. 28, 371
- **R. Badugu ; J. R. Lakowicz ; C. D. Geddes.** *Analyst,* 2004, vol. 129, 516
- **J. P. Lorand ; J. O. Edwards.** *J. Am. Chem. Soc.,* 1959, vol. 24, 769
- **Kikuchi A ; Suzuki K ; Okabayashi O ; Hoshino H ; Kataoka K ; Sakurai Y ; Okano T.** Glucose-sensing electrode coated with polymer complex gel containing phenylboronic acid. *Analytical Chemistry,* 1996, vol. 68, 823-828
- **G. J. Worsley ; G. A. Tourniaire ; K. E. S. Medlock ; F. K. Sartain ; H. E. Harmer ; M. Thatcher ; A. M. Horgan ; J. Pritchard.** *Clinical Chem.,* 2007, vol. 53, 1820-1826
- **S. Tanna ; T. S. Sahota ; J. Clark ; M. J. Taylor.** *J. Drug Target.,* 2002, vol. 10, 411
- **S. Tanna ; T. Sahota ; J. Clark ; M. J. Taylor.** *J. Pharm. Pharmacol.,* 2002, vol. 54, 1461
- **Yang XP ; Lee MC ; Sartain F ; Pan XH ; Lowe CR.** Designed boronate ligands for glucose-selective holographic sensors. *Chemistry-a European Journal,* 2006, vol. 12, 8491-8497
- **Tanna S ; Sahota T ; Clark J ; Taylor MJ.** A covalently stabilised glucose responsive gel formulation with a Carbopol (R) carrier. *Journal of Drug Targeting,* 2002, vol. 10, 411-418
- **Tanna S ; Sahota T ; Clark J ; Taylor MJ.** Covalent coupling of concanavalin A to a Carbopol 934P and 941 P carrier in glucose-sensitive gels for delivery of insulin. *Journal of Pharmacy and Pharmacology,* 2002, vol. 54, 1461-1469
- **Beckert WH ; Sharkey MM.** Mitogenic Activity of Jack Bean (Canavalia-Ensiformis) with Rabbit Peripheral Blood Lymphocytes. *International Archives of Allergy and Applied Immunology,* 1970, vol. 39, 337
- **Katoa N ; Gehrke SH.** Microporous, fast response cellulose ether hydrogel prepared by freeze-drying. *Colloids and Surfaces B: Biointerfaces,* 2004, vol. 38, 191-196

- **Kim JJ ; Park K.** Modulated insulin delivery from glucose-sensitive hydrogel dosage forms. *Journal of Controlled Release,* 2001, vol. 77, 39-47
- **M. Tang RZ ; A. Bowyer ; R. Eisenthal ; J. Hubble.** A reversible hydrogel membrane for controlling the delivery of macromolecules. *Biotechnology and Bioengineering,* 2003, vol. 82, 47-53
- **Eddington DT ; Beebe DJ.** A valved responsive hydrogel microdispensing device with integrated pressure source. *Journal of Microelectromechanical Systems,* 2004, vol. 13, 586-593
- **Zhao, B. ; Brittain, W. J.** *J. Am. Chem. Soc.,* 1999, vol. 121, 3557-3558
- **Hussemann, M. ; Malmstrom, E. ; McNamara, M. ; Mate, M. ; Mecerreyes, D. ; Benoit, D. G. ; Hedrick, J. L. ; Mansky, P. ; Huang, E. ; Russel, T. P.** *Macromolecules,* 1999, vol. 32, 1424-1431
- **Matyjaszewski, K. ; Miller, P. J. ; Shukla, N. ; Immaraporn, B. ; Gelman, A. ; Luokala, B. B. ; Siclovan, T. M. ; Kickelbick, G. ; Vallant, T. ; Hoffman, H.** *Macromolecules,* 1999, vol. 32, 8716-8724
- **Ejaz, M. ; Yamamoto, S. ; Ohno, K. ; Tsuji, Y. ; Fukuda, T.** *Macromolecules,* 1998, vol. 31, 5934-5926
- **von Werne, T. ; Patten, T. E.** *J. Am. Chem. Soc.,* 1999, vol. 121, 7409-7410
- **Sun, L. ; Baker, G. L. ; Bruening, M. L.** *Macromolecules,* 2005, vol. 38, 2307-2314
- **Shah, R. R. ; Merreceyes, D. ; Husemann, M. ; Rees, I. ; Abbott, N. L. ; Hawker, C. J. ; Hedrick, J. L.** *Macromolecules,* 2000, vol. 33, 597-605
- **Kim, J.-B. ; Bruening, M. L. ; Baker, G. L.** *J. Am. Chem. Soc.,* 2000, vol. 122, 7616-7617
- **Bruening, M. L. ; Baker, G. L.** *Macromolecules,* 2002, vol. 35, 1175-1179
- **Fan, X. ; Lin, L. ; Dalsin, J. L. ; Messersmith, P. B.** *J. Am. Chem. Soc.,* 2005, vol. 127, 15843-15847
- **Zhang, F. ; Xu, F. J. ; Kang, E. T. ; Neoh, K. G.** *Ind. Eng. Chem. Res.,* 2006, vol. 45, 3067-3073
- **Li, G. ; Fan, J. ; Jiang, R. ; Gao, Y.** *Chem. Mater.,* 2004, vol. 16, 1835-1837
- **Carlmark, A. ; Malmström, E.** *J. Am. Chem. Soc.,* 2002, vol. 124, 900-901
- **Roy, D. ; Knapp, J. S. ; Guthrie, J. T. ; Perrier, S.** *Biomacromolecules,* 2008, vol. 9, 91-99
- **Yao, F. ; Fu, G.-D. ; Zhao, J. P. ; Kang, E.-T. ; Neoh, K. G.** *J. Membr. Sci.,* 2008, vol. 319, 149-157
- **Yamamoto, K. ; Miwa, Y. ; Tanaka, H. ; Sakaguchi, M. ; Shimada, S.** *J. Polym. Sci., Part A: Polym. Chem.,* 2002, vol. 40, 3350-3359
- **Desai, S. M. ; Solanky, S. S. ; Mandale, A. B. ; Rathore, K. ; Singh, R. P.** *Polymer,* 2003, vol. 44, 7645-7649
- **Huang, J. Y. ; Murata, H. ; Koepsel, R. R. ; Russell, A. J. ; Matyjaszewski, K.** *Biomacromolecules,* 2007, vol. 8, 1396-1399
- **Barner, L. ; Perera, S. ; Sandanayake, S. ; Davis, T. P.** *J. Polym. Sci., Part A: Polym. Chem.,* 2006, vol. 44, 857-864
- **Kiani, K. ; Hill, D. J. T. ; Rasoul, F. ; Whittaker, M. ; Rintoul, L.** *J. Polym. Sci., Part A: Polym. Chem.,* 2007, vol. 45, 1074-1083
- **Yamamoto, K. ; Tanaka, H. ; Sakaguchi, M. ; Shimada, S.** *Polymer,* 2003, vol. 44, 7661-7669
- **J. Xingju ; Z. Xinge ; W. Zhongming ; T. Dayong ; Z. Xuejiao ; W. Yanxia ; W. Zhen ; L. Chaoxing.** *Biomacromolecules,* 2009, vol. 10, 1337-1345
- **L. Ionov ; N. Houbenov ; A. Sidorenko ; M. Stamm ; S. Minko.** *Adv. Funct. Mater.,* 2006, vol. 16, 1153-1160
- **A. Constable ; W. Brittain.** *Colloids and Surfaces a-Physicochemical and Engineering Aspects,* 2007, vol. 308, 123-128